# EUROPEAN PATENT APPLICATION

(11) **EP 4 477 294 A1**
(43) Date of publication of application: **18.12.2024**
(21) Application number: 23179663.2
(22) Date of filing: 16.06.2023
(51) Int. Cl.: B01D 53/58, B01D 53/14, B01D 53/62, C07D 251/60, F28D 7/00, B01D 53/54, B01D 53/78, F28F 9/013, F28F 9/02, F28F 9/22

(54) **OFFGAS WASHING SECTION OF A MELAMINE PLANT**

(71) Applicant: CASALE SA, 6900 Lugano (CH)
(72) Inventor: SCOTTO, Andrea, 6932 Breganzona (CH); GAMBA, Simone, 24040 Pagazzano (BG) (IT); DI CARLO, Gabriele, 6900 Lugano (CH)
(74) Representative: M. Zardi & Co S.A.

(57) **Abstract**

A melamine offgas washing section (100) wherein melamine offgas (3) is washed with urea melt in a scrubber (101) and the urea melt (9) collected from the scrubber after washing is partly recirculated to the scrubber, wherein the recirculated urea melt is cooled in a urea melt cooler (104) external to the scrubber, wherein the urea melt cooler is a floating-head shell-and-tube apparatus.

## Description

### Field of application

The invention is in the field of industrial production of melamine. The invention particularly pertains to an offgas washing section configured for washing melamine offgas with urea melt.

### Prior art

The technology for production of melamine at an industrial scale includes the non-catalytic high-pressure (HP) process and the low-pressure (LP) catalytic process. The non-catalytic high-pressure process is considered the most advantageous and is becoming predominant.

In the high-pressure process, a urea melt is reacted at pressure which is generally above 70 bar, typically 75 to 200 bar. The temperature of reaction is typically around 375 °C.

The melamine-containing product stream is sent to further treatments, typically performed at a lower pressure. Said treatments may include quenching, purification, crystallization, solid-liquid separation and drying, to obtain solid melamine of a desired purity. Typically, melamine purification and crystallization are carried out in an alkaline environment and ammonia or sodium hydroxide are the most commonly used alkaline agents.

The conversion of urea to melamine produces a gaseous stream ("melamine offgas") containing predominantly ammonia and carbon dioxide, some melamine, and other minor components.

It is generally desired to remove melamine from the offgas, to recover melamine and to purify the offgas for a further use, such as recycling the offgas as a feed material for a tied-in urea plant. A known technique to purify the melamine offgas is washing with urea melt in a suitable scrubber. A portion of the available urea melt feed can be used to this purpose, and the urea melt after washing can be sent to the melamine synthesis section.

US 7,311,759 discloses a scrubbing process of the melamine offgas wherein part of the urea melt withdrawn from the scrubber is recirculated to the same scrubber after cooling in an external heat exchanger ("urea melt cooler"). Said heat exchanger is typically a shell-and-tube heat exchanger where the urea melt traverses the tube side. The shell side is typically fed with water to produce steam. Particularly when the cooling medium is a boiling medium (most commonly water converted to steam), it is highly preferred that boiling takes place in the shell side for reasons of stability of the boiling process. Moreover, according to common design practices, the fluid at higher pressure or dirtier is preferably directed to the tube side of the heat exchanger. Due to the nature of the fluid to be cooled (urea melt), the cooling process, even when performed tube side, has some drawbacks.

The urea melt withdrawn from the scrubber contains melamine precursors and ammonia, as a result of the washing process in contact with the offgas. Due to its composition, the urea melt can leave unwanted deposits of solid material on the inner surface of the tubes, forming a layer of fouling or leading to occlusion of the tubes. The fouling causes degradation of the heat exchange coefficient and increase of flow resistance (pressure drop). The performance of the heat exchanger is seriously affected. Additionally, in case of excessive cooling of melamine melt, e.g. due to deviation from the normal operation, precipitation of melamine cyanurate may occur in the tubes.

### Summary of the invention

The invention faces the problem of how to increase reliability and how to maintain a constant performance of the above-identified heat exchanger (urea melt cooler) of a melamine offgas washing section.

The problem is solved with an offgas washing section according to the claims. The invention judiciously uses a floating-head heat exchanger as the urea melt cooler.

Said floating-head shell-and-tube heat exchanger has a bundle of straight tubes connected to two tubesheets at opposite ends. One tubesheet is fixed to the shell whereas the other tubesheet ("floating tubesheet") can move relative to the shell.

The invention comes from the understanding that tubes traversed by the urea melt, as in the case of interest, can be properly cleaned only by mechanical cleaning, also termed drilling, which requires access to the tubes. Therefore, a bundle of U-tubes is not advantageous in the present case. U-tubes, particularly the U-bend, cannot be cleaned mechanically by drilling. This is contrary to common design practice suggesting U-tubes as a measure to avoid an expansion joint on the shell side, particularly in a kettle-type heat exchanger.

The invention also comes from the understanding that alternative cleaning methods, available when the tubes are not accessible for drilling, are not effective against deposits caused by the urea melt after offgas washing. These methods include introduction of chemical agents, water jets at high pressure, or increasing the shell side temperature to liquefy the deposits.

A deposit of melamine cyanurate cannot be removed by heating. Experience shows that heating may lead to formation of polycondensates even more difficult to remove that the cyanurate itself. Washing with known chemical agents is not effective; basic or acid solutions or organic solvents have proven not effective to remove the cyanurate. Also washing with water is not satisfactory, the cyanurate being hard and insoluble in water. In particular, chemical cleaning or water washing are totally ineffective in case of tube clogging which does not allow the free flowing of the washing fluid.

The advantage of the invention is that the floating-head heat exchanger allows access to the inside of tubes for removing deposits and possible occlusion of tubes directly by means of a mechanical action such as tube drilling. Any deposit of melamine cyanurate can be separated from the metal surface by drilling and then removed with a water washing.

Another noticeable advantage of a floating-heat heat exchanger is it does not require an expansion joint of the shell. An expansion joint is structurally a weak point and is expensive. The art of designing heat exchangers suggests that an expansion joint can be avoided by using U-tubes but, as stated above, U-tubes are found not advantageous in the present application (urea melt cooler of an offgas scrubber) due to possible formation of deposits difficult to clean or remove.

Another aspect of the invention is a melamine plant including a melamine synthesis section, where urea melt is converted to melamine and an offgas comprising ammonia and carbon dioxide is formed, and further including an offgas washing section according to the claims, the offgas washing section being arranged to receive the offgas from the synthesis section. Still another aspect of the invention is a process for the synthesis of melamine according to the attached claims.

### Description of the invention

The invention related to a melamine offgas washing section arranged to process offgas containing ammonia and carbon dioxide emerging from a melamine synthesis section where urea is reacted to form melamine.

Said offgas washing section includes a scrubber arranged to provide washing of the offgas with urea melt. The scrubber is connected to a feed line of fresh urea melt. The washing section includes a line arranged to collect a stream of urea melt effluent from the scrubber after the washing process; a recirculation line external to the scrubber and arranged to reintroduce into the scrubber part of said effluent urea melt; a heat exchanger arranged in the recirculation line to cool the urea melt by transferring heat to a cooling medium. Accordingly, the scrubber receives a urea melt stream including fresh urea melt and recirculated urea melt.

Said heat exchanger is a floating-head shell-and-tube equipment. In general terms, said heat exchanger includes a tube bundle contained in a shell. The heat exchanger has first inlet/outlet connections for the tube bundle and second inlet/outlet connections for the shell. Accordingly, the heat exchanger has a tube side and a shell side which can be traversed, respectively, by a first medium and a second medium. In the present invention, the heat exchanger is connected so that the recirculated urea melt traverses the tube side and a cooling medium traverses the shell side.

The tubes are disposed straight from a first tubesheet to a second tubesheet. According to the principle of floating head, the heat exchanger has a fixed tubesheet and a floating tubesheet. The fixed tubesheet is fixed to a flange whereas the floating tubesheet is free to move, for example following thermal expansion of the tubes. Conventionally, the fixed tubesheet is considered the front-end of the heat exchanger and the floating tubesheet is considered the rear end thereof.

Preferred embodiments are described with the aid of the nomenclature of TEMA (Tubular Exchanger Manufacturers Association). The TEMA symbols are familiar to a skilled person and are summarized here.

### Front end stationary head types

- A: Channel and removable cover
- B: Bonnet (integral cover)
- C: Channel integral with tubesheet and removable cover; removable tube bundle
- N: Channel integral with tubesheet and removable cover; tube bundle not removable
- D: Special high-pressure closure

### Shell types

- E: One pass shell
- F: Two pass shell with longitudinal baffle
- G: Split flow
- H: Double split flow
- J: Divided flow
- K: Kettle type
- X: Cross flow

### Rear end head types

- L: Fixed tubesheet, like "A" stationary head
- M: Fixed tubesheet, like "B" stationary head
- N: Fixed tubesheet, like "N" stationary head
- P: Outside packed floating head
- S: Floating head with backing device
- T: Pull through floating head
- U: U-tube bundle
- W: Externally sealed floating tubesheet

In the embodiments of the invention, the shell may be of types E, F, G, H, J, K or X according to TEMA. The front end may be of types A, B, C, N, D. The rear-end head is a floating head and, accordingly, may be of P, S, T or W type.

The symbols can be combined. A three-letter code identifies, in the order, the type of the front head, the type of the kettle and the type of the rear head. For example, "CKT" denotes a heat exchanger where the front-end head is a stationary head according to "C" type; the shell is according to "K" type; the rear end head is a floating head is according to the "T" type. Various embodiments of the invention include combinations of the above types of front-end, shell and rear-end.

Details of the construction of heat exchangers, and related nomenclature, need not be explained as they can be found in the literature, such as Perry's Chemical Engineers' Handbook, 7th Edition, Chapter 11, "Heat Transfer Equipment".

In a preferred embodiment, said heat exchanger has multiple passages of the tube side. Preferably the number of passages of the tube side is an even number, particularly preferably the number of passages is two, four or six.

Preferably, the tubes of the heat exchanger are seamless tubes. Seamless tubes increase reliability and resistance to corrosion. Preferred materials for the tubes include nickel alloys or titanium.

The front-end of the heat exchanger is preferably a Type-C or Type-N stationary head according to TEMA designation.

In an embodiment, the tube bundle is fully removable from the shell of the heat exchanger to allow cleaning in a separate location.

The heat exchanger may be a kettle-type heat exchanger, preferably a CKT exchanger according to TEMA designation.

The heat exchanger may include a manhole adapted to provide access to the tube side for cleaning of tubes. The provision of such manhole is particularly preferred in combination with a kettle-type heat exchanger.

The cooling medium in the shell side may be any suitable medium. In some embodiments the cooling medium is at least partly evaporated in the shell side. The preferred cooling medium is water and, in a preferred embodiment, water is evaporated in the shell side to produce steam. Accordingly the shell side has an inlet connected to a water feeding line and an outlet connected to a steam pipe.

In some embodiments, a condensate stream is purged from the shell side of the heat exchanger, to control the concentration of salts in the water. The amount of the purged condensate is preferably not greater than 100 kg/h.

The urea melt is preferably cooled to a temperature in the range 165 to 245 °C. A temperature of the urea melt above 165 °C avoids the precipitation of the cyanurate, whereas a temperature not greater than 245 °C is beneficial to avoid corrosion, i.e. to control the corrosive effect of the urea melt flowing through the urea melt cooler.

The non-recirculated portion of the urea melt can be sent to the melamine synthesis section.

The scrubber may be single-stage or may include two stages. The contact between the melamine offgas and the urea melt is preferably counter-current. Preferably, the scrubber is arranged vertically.

In a two-stage embodiment, the scrubber includes a first stage and a second stage. In the first stage the melamine offgas is contacted in counter-current direction with the urea melt comprising the recirculated urea melt loaded with ammonia and melamine precursors; in the second stage the offgas emerging from the first stage is contacted in counter-current with a fresh urea melt, said fresh urea melt being introduced in the second stage and traversing in sequence the second stage and the first stage. The two stages operate in sequence, so that the partially purified offgas effluent from the first stage is processed in the second stage. The first stage can be operated at a higher temperature than the second purification step, or the two steps may be performed substantially at the same temperature.

A preferred embodiment of a two-stage scrubber is as follows. The second purification stage is placed above the first purification stage; the melamine offgas flows upward in the first stage and then in the second stage; a urea melt is sprayed over the offgas from top of the second stage and flows downward through the second stage and the first stage; a urea melt loaded with ammonia and melamine precursors is collected at the bottom of the first stage and a portion thereof is recirculated to the same first stage after cooling. The melamine offgas is scrubbed in counter-current firstly with the urea melt from the second stage and the urea melt recirculated in the first stage; then with the fresh urea melt introduced in the second stage. Preferably the first stage operates in a temperature range of 170 °C to 250 °C, whereas the second stage operates at 135 °C to 230 °C.

In some embodiments, the offgas washing includes the addition of carbon dioxide. A carbon dioxide stream can be added to the melamine offgas before it enters the scrubber, or can be introduced separately into the scrubber. In an embodiment with two stages, addition of carbon dioxide is made preferably to the first stage.

The washing of the melamine offgas with urea melt is performed at a high pressure, preferably of at least 50 bar and more preferably equal to or substantially equal to the melamine synthesis pressure. The offgas washing may be performed at a pressure slightly less than the melamine synthesis pressure, wherein the difference is not more than 20 bar or not more than 5 bar. The purified offgas after washing may be recycled to a tied-in urea plant.

According to an embodiment, the off-gas is introduced in the scrubber via an off-gas distributor above or below a liquid level of the urea melt containing ammonia and melamine precursors. A preferred embodiment of said offgas distributor is disclosed in US 7,311,759. The urea melt may be introduced in the scrubber as a single stream or divided into a plurality of streams and introduced at multiple locations of the scrubber, e.g. at different vertical elevation.

The fresh urea melt is preferably a urea melt obtained from a urea plant after recovery of unreacted matter and evaporation of water. Typically, the urea melt contains at least 96% urea, the balance being residual water and unavoidable impurities.

In the melamine synthesis section, the urea melt is reacted under non-catalytic high pressure melamine synthesis conditions to generate a raw melamine product and a stream of melamine offgas comprising ammonia, carbon dioxide, melamine and minor components. The melamine synthesis pressure is preferably 70 bar or above, for example 70 bar to 200 bar.

According to a preferred embodiment, the synthesis of melamine includes a conversion step and a stripping step, wherein said conversion step includes reacting said urea melt feed stream under suitable melamine synthesis conditions to generate a raw melamine product, and said stripping step includes the stripping of said raw melamine product in the presence of gaseous ammonia, to remove carbon dioxide contained in the raw melamine.

In some embodiments, the melamine synthesis section includes a single reactor, from which the raw melamine and the melamine offgas are withdrawn. In other embodiments, said melamine synthesis section includes a primary reactor where urea melt is reacted, followed by a secondary reactor where the melamine-containing effluent of the primary reactor is stripped with gaseous ammonia. In such embodiments, each of the primary reactor and the secondary reactor produce a respective stream of melamine offgas. Both melamine offgas streams are made predominantly of ammonia and carbon dioxide, although they may differ in composition.

The melamine offgas subject to scrubbing with urea melt, in accordance with embodiments the invention, may include only the melamine offgas stream from the primary reactor or both melamine offgas streams from the primary reactor and secondary reactor, possibly combined into a single stream. In a further embodiment, a combined reactor performs the function of the primary reactor and secondary reactor; to this purpose, said combined reactor includes a primary reaction stage and a secondary reaction stage.

The melamine-contained effluent of the synthesis section is processed in a melamine purification stage according to known technique. The capacity of the melamine synthesis process is typically 5 to 15 tons of melamine per hour, said capacity being the amount solid melamine that is obtained from the process.

In a combined urea-melamine embodiment, ammonia and carbon dioxide are reacted to form a urea solution in a urea synthesis section, the urea solution is processed in at least one recovery section to obtain a purified urea solution and water is removed from the solution to form a urea melt. Said urea melt is used in the above-described process for synthesis of melamine. The melamine offgas generated during the synthesis of melamine is recycled to the production of urea.

### Description of the figures

Fig. 1 is a scheme of a melamine offgas scrubbing section.
Fig. 2 is a scheme a urea melt cooler that can be used in the scrubbing section, in accordance with an embodiment of the present invention.
Fig. 3 is a scheme of a urea melt cooler in accordance with another embodiment.

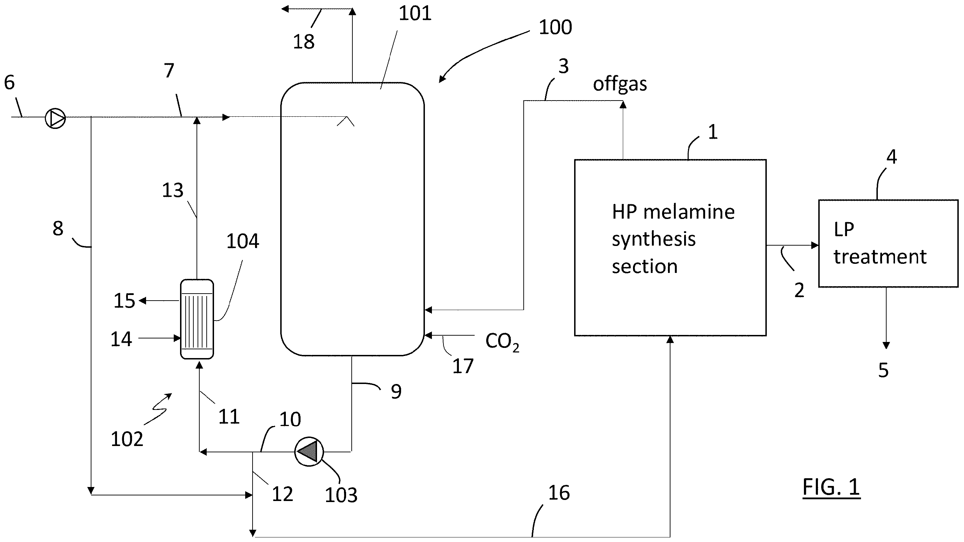

The main items in in Fig. 1 are:
- 1: melamine synthesis section
- 2: raw melamine melt effluent
- 3: offgas withdrawn from the melamine synthesis section ("melamine offgas")
- 4: low-pressure (LP) treatment section of the raw melamine melt
- 5: solid melamine
- 100: melamine offgas washing section
- 101: offgas scrubber
- 102: urea melt recirculation line
- 103: urea melt recirculation pump
- 104: urea melt cooler
- 6: fresh urea melt from a urea plant
- 7: fresh urea melt input to the scrubber 101
- 8: fresh urea melt directed to the melamine synthesis section 1
- 9: urea melt after the scrubbing process
- 10: urea melt delivered by the pump 103
- 11: urea melt recirculated to the scrubber 101
- 12: urea melt sent to the synthesis section 1
- 13: cooled urea melt
- 14: water
- 15: steam
- 16: urea melt feed to the synthesis section 1
- 17: CO2 feed to the scrubber 101
- 18: purified offgas removed from the scrubber 101

The reference numbers are intended to denote process streams as well as corresponding connections lines.

The urea melt feed 16 is reacted in the melamine synthesis section 1 to produce the melamine-containing product 2 and melamine offgas 3.

The melamine offgas 3 are treated in the washing section 101 to obtain purified offgas 18 which may be recycled to a tied-in urea plant. Particularly, the melamine offgas 3 are washed with urea melt in the scrubber 101. Offgas washing is performed with fresh urea melt 7 and recycled urea melt 13 previously cooled in the urea melt cooler 104. The scrubber 101 may be single-stage or double-stage. Washing is performed in a counter-current in the scrubber 101, possibly with the aid of carbon dioxide 17.

The recirculation line 102 includes the urea melt pump 103 and the urea melt cooler 104. The recycled urea melt 11 is part of the urea melt 9 withdrawn from the scrubber 101. The remaining part 12 is added to the portion 8 of the fresh urea melt to form the feed 16 of the melamine synthesis section 1.

In the urea melt cooler 104, heat is removed from the recycled urea melt 11 and transferred to water 14, which is converted to steam 15. The urea melt cooler 104 is a shell-and-tube equipment where the urea melt 11 is fed to the tube side and evaporation of the water 14 takes place in the shell side.

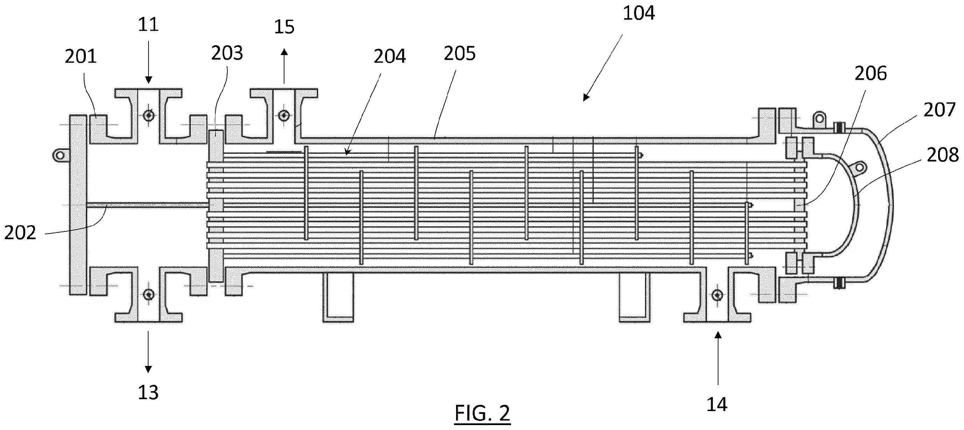

An embodiment for the urea melt cooler 104 is illustrated in Fig. 2:
- 201: stationary-head channel
- 202: pass partition
- 203: stationary tubesheet
- 204: tube bundle
- 205: shell
- 206: floating tubesheet
- 207: shell cover
- 208: floating-head cover.

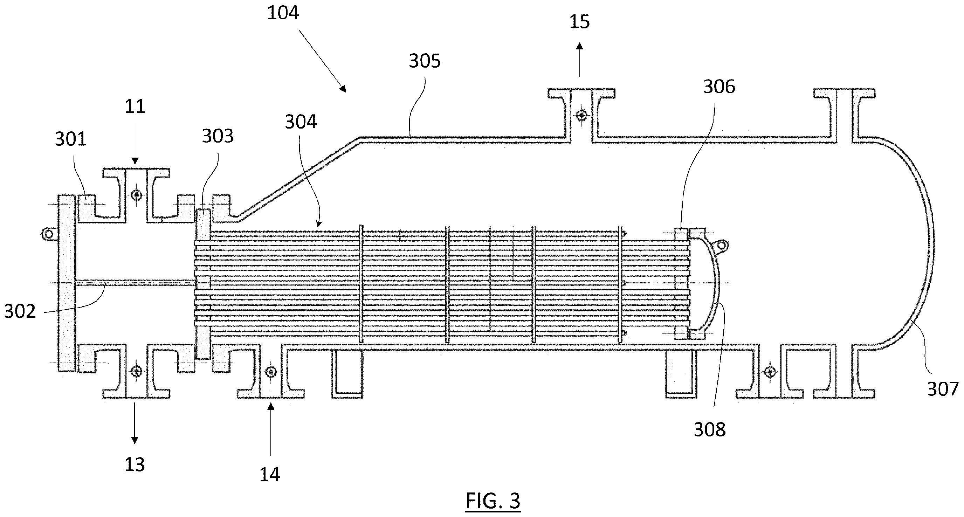

Fig. 3 illustrates an alternative embodiment of kettle type:
- 301: stationary-head channel
- 302: pass partition
- 303: stationary tubesheet
- 304: tube bundle
- 305: shell
- 306: floating tubesheet
- 307: shell cover
- 308: floating-head cover.

## Claims

1. A melamine offgas washing section (100) arranged to process offgas (3) containing ammonia and carbon dioxide emerging from a melamine synthesis section (1), wherein said offgas washing section includes:
a scrubber (101) connected to a urea melt feed line, said scrubber being arranged to provide washing of the offgas with urea melt;
a line (9) arranged to collect a stream of urea melt effluent from the scrubber after the washing process;
a recirculation line (102) external to the scrubber and arranged to reintroduce into the scrubber part (11) of said effluent urea melt;
a heat exchanger (104) arranged in the recirculation line to cool the recirculated urea melt (11) by transferring heat to a cooling medium;
said heat exchanger (104) being a shell-and-tube equipment with a tube bundle contained in a shell, the tube bundle and the shell having inlet and outlet connections arranged so that the recirculated urea melt (11) traverses the tube side and the cooling medium (14) traverses the shell side;
said heat exchanger (104) being a floating-head heat exchanger.

2. A melamine offgas washing section according to claim 1 wherein said heat exchanger has multiple passages of the tube side.

3. A melamine offgas washing section according to claim 2 wherein the number of passages of the tube side is an even number, preferably two, four or six passages.

4. A melamine offgas washing section according to any of the previous claims wherein the tubes of the heat exchanger are seamless tubes.

5. A melamine offgas washing section according to any of the previous claims wherein said heat exchanger has a front-end with a Type-C or Type-N stationary head according to TEMA designation.

6. A melamine offgas washing section according to claim 5 wherein the tube bundle is fully removable from the shell of the heat exchanger to allow cleaning in a separate location.

7. A melamine offgas washing section according to any of the previous claims wherein said heat exchanger is a kettle-type heat exchanger, preferably a CKT exchanger according to TEMA designation.

8. A melamine offgas washing section according to claim 7 wherein the heat exchanger includes a manhole adapted to provide access to the tube side for cleaning of tubes.

9. A melamine offgas washing section according to any of the previous claims wherein the heat exchanger is configured to produce steam (15) in the shell side, the shell side having an inlet connected to a water feeding line and an outlet connected to a steam pipe.

10. A melamine plant including a melamine synthesis section, where urea melt is converted to melamine and an offgas comprising ammonia and carbon dioxide is formed, and an offgas washing section according to any of the previous claims, the offgas washing section being arranged to receive the offgas from the synthesis section.

11. A process for the synthesis of melamine including the steps of:
reacting a feed stream of urea melt under non-catalytic high pressure melamine synthesis conditions to generate a raw melamine product (2) and an offgas (3) comprising ammonia and carbon dioxide,
washing the offgas (3) with urea melt in a scrubber (101), wherein a stream of urea melt (9) containing ammonia and melamine precursors, resulting from the washing process, is withdrawn from the scrubber;
wherein a portion (11) of the urea melt (9) withdrawn from the scrubber is recycled to the same scrubber via a recirculation line (102) external to the scrubber;
said recirculation line including a heat exchanger (104) arranged to cool the recirculated urea melt (11) by transferring heat to a cooling medium;
said heat exchanger (104) being a shell-and-tube equipment with a tube side and a shell side, wherein the urea melt traverses the tube side and the cooling medium is fed to the shell side;
said heat exchanger (104) being a floating-head heat exchanger.

12. A process according to claim 11, the floating-head heat exchanger being in accordance with any of claims 2 to 9.

13. A process according to claim 11 or 12 wherein the cooling medium is water which is converted to steam in the shell side of said heat exchanger.

14. A process according to claim 13, wherein a condensate stream is purged from the shell side of the heat exchanger, to control the concentration of salts in the water, and the amount of the purged condensate is not greater than 100 kg/h.
